Europäisches Patentamt

European Patent Office

Office européen des brevets

(19)

(11) Numéro de publication: **0 194 201**
**A1**

## (12) DEMANDE DE BREVET EUROPEEN

(21) Numéro de dépôt: **86400437.9**

(22) Date de dépôt: **03.03.86**

(51) Int. Cl.⁴: **C07C 139/04**

(30) Priorité: **04.03.85 FR 8503116**

(43) Date de publication de la demande:
**10.09.86 Bulletin 86/37**

(84) Etats contractants désignés:
**BE DE FR GB IT NL**

(71) Demandeur: **DUMAS ET INCHAUSPE**
**La Traversière Route de Gouze**
**F-64370 Arthez de Bearn(FR)**

(72) Inventeur: **Naon, Hubert**
**8 boulevard des Pyrénées**
**F-64000 Pau(FR)**

(74) Mandataire: **Lepeudry-Gautherat, Thérèse et al**
**ARMENGAUD JEUNE CABINET LEPEUDRY 23**
**boulevard de Strasbourg**
**F-75010 Paris(FR)**

(54) Prefectionnement au procédé pour la photosulfoxydation des N-paraffines et des esters gras méthyliques.

(57) Perfectionnement au procédé de photosulfoxydation de N-paraffines ou d'esters gras méthyliques.

Selon ce perfectionnement on ajoute quelques pour cent molaires d'une alkylamine éventuellement hydroxylée, dont chaque radical alkyle contient au plus quatre atomes de carbone.

Application en photosulfoxydation.

EP 0 194 201 A1

Perfectionnement au procédé pour la photosulfoxydation des N-paraffines et des esters gras méthyliques.

La présente invention concerne un perfectionnement au procédé pour la photosulfoxydation des N-paraffines et des esters gras méthyliques.

Actuellement, selon le procédé industriel connu pour la photosulfoxydation des N-paraffines, on fait réagir un mélange gazeux d'anhydride sulfureux et d'oxygène avec une N-paraffine émulsionnée dans l'eau, l'ensemble étant soumis à une irradiation ultra-violette. Un des inconvénients d'un tel procédé est que l'on produit simultanément de l'acide sulfurique et de l'acide sulfonique :

le produit brut de la réaction de la photosulfoxydation contient, en poids, une partie d'acide sulfurique pour trois parties d'acide sulfonique. Il faut donc éliminer l'acide sulfurique, ce qui fait appel à des techniques longues et coûteuses.

La réaction de photosulfoxydation des N-paraffines peut également être réalisée dans un milieu anhydre. Il est produit beaucoup moins d'acide sulfurique que précédemment, mais, par contre, le produit de réaction peut contenir jusqu'à 5 % en poids d'ions sulfate : ceci est excessif, d'où la nécessité de prévoir une étape de purification ultérieure. De plus, il se forme des dépôts noirâtres sur les gaines de verre et les parois du photoréacteur qui rendent difficile, voire même impossible, un fonctionnement en continu de la réaction de photosulfoxydation.

Les différents problèmes soulevés par la photosulfoxydation sont particulièrement analysés dans un article de C. Beermann dans European Chemical News, Normal Paraffins supplement, December 2, 1966.

Dans le brevet des Etats-Unis d'Amérique n° 3 682 803, il est indiqué que la formation de dépôts noirâtres peut être supprimée en n'utilisant que des radiations ultraviolettes dont la longueur d'onde est supé rieure à 300 nm. Or il a été constaté que de tels dépôts se formaient lorsqu'on utilise une lampe à vapeur de mercure basse pression émettant entre 300 et 500 nm avec un maximum à 365 nm, aucune radiation inférieure à 300 nm n'étant émise.

Aussi un des buts de la présente invention est-il un perfectionnement au procédé pour la photosulfoxydation de N-paraffines et d'esters gras méthyliques qui permet de supprimer la production concommittante d'acide sulfurique.

Un autre but de l'invention est un tel perfectionnement qui permet également de prévenir tout dépôt noirâtre.

Ces buts ainsi que d'autres qui apparaîtront par la suite, sont atteints par un perfectionnement au procédé pour la photosulfoxydation de N-paraffines ou d'esters gras méthyliques, selon lequel on fait réagir un mélange gazeux d'anhydride sulfureux et d'oxygène avec un milieu comprenant des N-paraffines ou des esters gras méthyliques en présence d'une irradiation ultraviolette, et au cours duquel, selon la présente invention, on ajoute quelques pourcents molaires d'une alkylamine éventuellement hydroxylée dont chaque radical alkyle contient au plus quatre atomes de carbone.

De préférence, la quantité d'alkylamine ajoutée est proportionnelle à celle d'acide sulfonique formé dans le milieu réactionnel, le rapport des concentrations molaires alkylamine/acide sulfonique étant compris entre 0,75 et 1,75.

Avantageusement, lorsque le milieu réactionnel comprend des N-paraffines de pétrole, le rapport des concentrations molaires alkylamine/acide sulfonique est compris entre 1,3 et 1,5.

De préférence l'alkylamine est choisie dans le groupe constitué par la monoéthanolamine, la triéthanolamine, la triéthylamine et la tributylamine.

Avantageusement, l'alkylamine est la triéthanolamine.

La description qui va suivre et qui ne présente aucun caractère limitatif, est relative à un exemple de réalisation de la présente invention afin de permettre à l'homme du métier de mieux en comprendre les caractéristiques. Elle doit être lue en regard des figures annexées, parmi lesquelles :

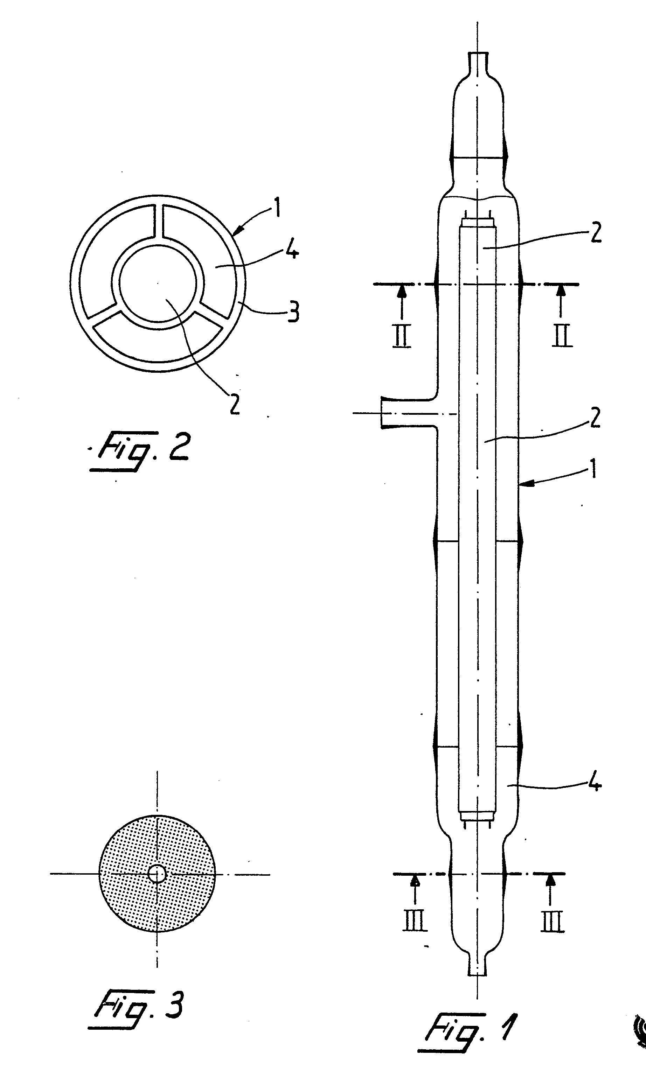

-la figure 1 représente en vue de face un réacteur dans lequel est réalisé l'invention ;

-la figure 2 est une coupe transversale selon la ligne II-II de la figure 1 ;

-la figure 3 est une coupe transversale selon la ligne III-III de la figure 1.

Pour réaliser une réaction de photosulfoxydation selon la présente invention, on utilise un photoréacteur désigné dans son ensemble par la référence 1. Il comprend en son milieu une lampe 2 à décharge de mercure basse pression comme source de radiations ultraviolettes. Cette lampe 2 émet la longueur d'onde de résonnance du mercure (265 nm), laquelle excite une poudre fluorescente la recouvrant : la lumière réémise a une longueur d'onde comprise entre 300 nm et 400 nm. Le spectre de cette réémission a la forme d'une cloche ayant un maximum à 365 nm.

L'enveloppe 3 du photoréacteur 1 est un tube de verre de 80 mm de diamètre, selon cet exemple de réalisation.

Le volume réactionnel est constitué par l'espace annulaire 4 qui est compris entre la lampe 2 et l'enveloppe 3 du photoréacteur 1. Pour obtenir un rapport surface/volume élevé, la distance annulaire, c'est-à-dire celle séparant la lampe 2 de l'enveloppe 3, est faible : 21,5 mm dans le présent exemple.

Le photoréacteur 1 est rempli d'un mélange de N-paraffines dont le nombre moyen d'atomes de carbone est voisin de quinze. On ajoute à ce mélange 3 % molaire d'une amine telle que la monoéthanolamine (MEA), la triéthanolamine (TEA), la triéthylamine (TEYA) et la tributylamine (TBA). Puis on injecte l'anhydre sulfureux gazeux ($SO_2$) qui est absorbé par le mélange et le colore légèrement en jaune. On additionne alors un courant d'oxygène à celui d'anhydride dans un rapport volumétrique $SO_2/O_2 = 2$.

A ce stade, on allume la lampe 2 pour démarrer la réaction que l'on laisse progresser pendant 180 minutes. La température de la réaction se stabilise à une valeur comprise entre 40 et 45°C, la température ambiante étant de 20°C. Puis on dégaze le milieu réactionnel pendant 90 mn à l'air. A la fin de cette expérience, on analyse les différents produits présents dans le milieu réactionnel. Dans le tableau I sont regroupés les résultats de ces analyses.

TABLEAU I

| Amine | sans | MEA | TEA | TEYA | TBA |
|---|---|---|---|---|---|
| Acide sulfonique (% en poids) | 9 | 9 | 8 | 3,5 | 3,5 |
| $SO_4=$(% en poids) | 9 | 5 | 1 | négligeable | négligeable |
| Dépôt noirâtre | beaucoup | peu | très peu | très peu | aucun |

*Les concentrations en acide sulfonique et en ion sulfate ont été mesuréses sur un échantillon de l'extrait obtenu en ajoutant de la monoéthanolamine au milieu selon le procédé décrit dans le document FR-A-2 516 079. La concentration en acide sulfonique a été mesurée par la méthode EPTON, et celle en ion sulfate directement par potentiométrie.

D'après ce tableau I, on constate que moins le milieu contient d'ions sulfate, c'est-à-dire d'acide sulfurique, moins il y a de dépôt noirâtre sur les parois du photoréacteur. Par ailleurs on remarque que la concentration en acide sulfonique produit est acceptable lorsque le milieu réactionnel comprend de la monoéthanolamine ou de la triétha nolamine mais que, par contre, elle est faible en présence de triéthylamine ou de tributylamine. Ceci serait dû à la solubilité partielle de ces deux amines dans les N-paraffines.

Dans une autre série d'expériences, le pourcentage en amine a été étudié. Ajouter plus de 3 % molaire d'amine n'améliore pas les résultats ci-dessus. Par contre si ce pourcentage est inférieur à 3 % molaire et devient notamment égal à 1 % molaire, on constate l'apparition de dépôt noirâtre et la présence d'acide sulfurique en quantité détectable.

Dans les expériences précédentes, l'amine est ajoutée à 3 % molaire au début, c'est-à-dire à un moment où la concentration en acide sulfonique est nulle, et aucune addition ultérieure n'a lieu. Pour simuler une photosulfoxydation continue, on a réalisé un essai au cours duquel l'amine, en l'occurence la triéthanolamine (TEA), a été ajoutée progressivement au milieu et de façon proportionnelle à la quantité d'acide sulfonique formé : les résultats de cet essai sont regroupés dans le tableau II, la quantité finale d'acide sulfonique produit étant de 6 % molaire.

TABLEAU II

| Quantité totale de TEA ajoutée en % molaire | 3 | 6 | 8,4 | 9 |
|---|---|---|---|---|
| Dépôt noirâtre | oui | oui | non | non |
| Phase lourde | oui | oui | non | non |
| Emulsion | non | partielle | totale | totale |
| Autodécantation | non | non | non | oui |

La phase lourde est due au sulfite non dissocié qui est créé par la combinaison de la triéthano lamine avec l'anhydride sulfureux.

On remarque que pour un rapport TEA/acide sulfurique égal à 1,4, on obtient une émulsion d'un blanc laiteux qui lave complètement les parois du photoréacteur 1. Cette émulsion apparaît dès le début de la réaction et se maintient jusqu'à la fin.

Cette émulsion est brisée par adjonction de monoéthanolamine pour extraire les produits acides selon le procédé décrit dans le document FR-A-2 516 079.

Bien que les essais réalisés ci-dessus aient eu lieu sur des N-paraffines, des résultàts analogues sont aussi obtenus en phososulfoxydant des esters gras méthyliques.

## Revendications

1. Perfectionnement au procédé de photosulfoxydation de N-paraffines ou d'esters gras méthyliques selon lequel on fait réagir un mélange gazeux d'anhydride sulfureux et d'oxygène avec un milieu comprenant des N-paraffines ou des esters gras méthyliques en présence d'une irradiation ultraviolette, caractérisé en ce qu'on ajoute quelques pour cent molaires d'une alkylamine éventuellement hydroxylée, dont chaque radical alkyle contient au plus quatre atomes de carbone.

2. Perfectionnement selon la revendication 1, caractérisé en ce que la quantité d'alkylamine ajoutée est proportionnelle à celle d'acide sulfonique formé dans le milieu réactionnel, le rapport des concentrations molaires alkylamine/acide sulfonique étant compris entre 0,75 et 1,75.

3. Perfectionnement selon la revendication 1 ou 2, caractérisé en ce que, lorsque ledit milieu réactionnel comprend des N-paraffines, ledit rapport est compris entre 1,3 et 1,5.

4. Perfectionnement selon l'une quelconque des revendications 1 à 3, caractérisé en ce que ladite alkylamine éventuellement hydroxylée dont chaque radical alkyle contient au plus quatre atomes de carbone, est choisie dans le groupe constitué par la monoéthanolamine, la triéthanolamine, la triéthylamine et la tributylamine.

5. Perfectionnement selon la revendication 4, caractérisé en ce que ladite alkylamine est la triéthanolamine.

_Fig. 2_

_Fig. 3_

_Fig. 1_

# RAPPORT DE RECHERCHE EUROPEENNE

Office européen des brevets

Numero de la demande

EP 86 40 0437

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 080 388 (DUMAS & INCHAUSPE) & FR - A - 2 516 079 (Cat. D) | | C 07 C 139/04 |
| A | BE-A- 701 667 (BAYER) | | |

DOMAINES TECHNIQUES RECHERCHES (Int. Cl.4)

C 07 C 139/00
C 07 C 143/00

Le présent rapport de recherche a été établi pour toutes les revendications

| Lieu de la recherche | Date d'achèvement de la recherche | Examinateur |
|---|---|---|
| LA HAYE | 13-05-1986 | VAN GEYT J.J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82